# EUROPEAN PATENT APPLICATION

(11) **EP 3 871 735 A1**
(43) Date of publication of application: **01.09.2021**
(21) Application number: 19877392.1
(22) Date of filing: 20.08.2019
(51) Int. Cl.: A61N 5/06, H01L 51/50, H05B 33/02

(54) **ORGANIC ELECTRIC FIELD LIGHT-EMITTING SHEET FOR USE IN LIGHT COSMETOLOGY OR LIGHT THERAPY**

(30) Priority: 26.10.2018 JP 2018201840
(71) Applicant: Nippon Shokubai Co., Ltd., Osaka-shi, Osaka 541-0043 (JP)
(72) Inventor: GOYA, Tsuyoshi, Suita-shi, Osaka 564-0034 (JP); KUWADA, Kenji, Suita-shi, Osaka 564-0034 (JP); MORII, Katsuyuki, Suita-shi, Osaka 564-0034 (JP)
(74) Representative: Cabinet Beau de Loménie
(86) International application number: PCT/JP2019/032341
(87) International publication number: WO 2020/084876

(57) **Abstract**

The present invention provides a light cosmetology or light therapy light-emitting sheet having excellent conformability to the skin and excellent wearing feeling. The present invention relates to a light cosmetology or light therapy organic electroluminescence sheet used in light cosmetology or light therapy application, the organic electroluminescence sheet including an organic electroluminescence device having a structure in which multiple layers are laminated on a sheet-like flexible substrate and having a thickness of 10 to 100 µm.

## Description

### TECHNICAL FIELD

The present invention relates to a light cosmetology or light therapy organic electroluminescence sheet. Specifically, the present invention relates to an organic electroluminescence sheet that applies light to the skin for cosmetic purposes or treatment of injuries and illnesses.

### BACKGROUND ART

Application of light to the skin has been used for cosmetic purposes or treatment of injuries and illnesses. For example, cosmetic instruments have been disclosed which apply light to the skin in order to remove blotches and wrinkles using the action of the light on cells (see Patent Literatures 1 and 2). Known cosmetic instruments which apply light include an instrument used near the skin and applies light as disclosed in Patent Literature 1 and a mask with light-emitting diodes used on the face as disclosed in Patent Literature 2, for example. The skin has irregularities, and the conditions of the skin are different among the parts. Thus, these instruments have difficulty in uniformly applying light to the skin and in controlling light application to match the skin conditions.

In response to such difficulty, the following products have been proposed: an ambulatory device which is adapted to conform to the surface of the area to be treated with light from the organic light-emitting semiconductor; a light therapy patch adapted to conform to a non-planar portion of a patient's body; and a sealing body for a biocompatible light-emitting device containing in a protective bag a biocompatible light-emitting device having a flexible substrate and a light-emitting device containing either an organic semiconductor or a quantum dot (see Patent Literatures 3 to 5). Also, a method has been proposed in which a cosmetic effect and a therapeutic effect are achieved by activating the skin by spreading a sheet containing an organic electroluminescent element on the skin and applying light (see Patent Literature 6, Non-Patent Literatures 1 and 2).

### CITATION LIST

### - Patent Literature

Patent Literature 1: JP 2013-154229 A
Patent Literature 2: JP 2005-27702 A
Patent Literature 3: JP 4651281 B
Patent Literature 4: WO 00/015296
Patent Literature 5: WO 17/038655
Patent Literature 6: JP 2015-142717 A

### - Non-Patent Literature

Non-Patent Literature 1: Yongmin Jeon and seven others, "Advanced Materials Technologies", 2018, 1700391
Non-Patent Literature 2: Tomoyuki Yokota and nine others, "Science Advances", 2016, Vol. 2, e1501856

### SUMMARY OF INVENTION

### - Technical Problem

As described above, various light-emitting apparatuses have been proposed for light cosmetology and light therapy applications. They are however insufficient in conformability to the skin with irregularities and in wearing feeling, and have room for improvement.

The present invention has been made in view of such a current state of the art and aims to provide a light cosmetology or light therapy light-emitting sheet having excellent conformability to the skin and excellent wearing feeling.

### - Solution to Problem

The present inventors examined light-emitting sheets having excellent conformability to the skin and excellent wearing feeling and found that use of an organic electroluminescence device having a structure in which multiple layers are laminated on a sheet-like flexible substrate and having a thickness of 10 to 100 µm as a light source provides a light-emitting sheet having excellent conformability to the skin and excellent wearing feeling.

The present inventors also found that use of, as an organic electroluminescence device, a so-called inverted organic electroluminescence device having a structure in which multiple layers are laminated between a cathode on a sheet-like flexible substrate and an anode provides an organic electroluminescence device-containing sheet having a long device lifetime. Thereby, the present invention has been completed.

That is, the present invention relates to a light cosmetology or light therapy organic electroluminescence sheet used in light cosmetology or light therapy application, the organic electroluminescence sheet including:
an organic electroluminescence device having a structure in which multiple layers are laminated on a sheet-like flexible substrate and having a thickness of 10 to 100 µm.

Preferably, the organic electroluminescence device includes a structure in which the multiple layers are laminated between a cathode on the sheet-like flexible substrate and an anode.

Preferably, the organic electroluminescence device includes a metal oxide layer between the cathode on the sheet-like flexible substrate and the anode.

Preferably, the organic electroluminescence sheet further includes a thin film battery.

Preferably, the organic electroluminescence sheet has a thickness of 650 µm or smaller.

The present invention also relates to a light cosmetology instrument including:
the light cosmetology or light therapy organic electroluminescence sheet of the present invention.

The present invention also relates to a light therapy instrument including:
the light cosmetology or light therapy organic electroluminescence sheet of the present invention.

### - Advantageous Effects of Invention

The light cosmetology or light therapy organic electroluminescence sheet of the present invention has high conformability to the skin and excellent wearing feeling and is thus suitable as a sheet which is attached for use to the skin and which applies light for cosmetic purposes and treatment of injuries and illnesses.

### BRIEF DESCRIPTION OF DRAWINGS

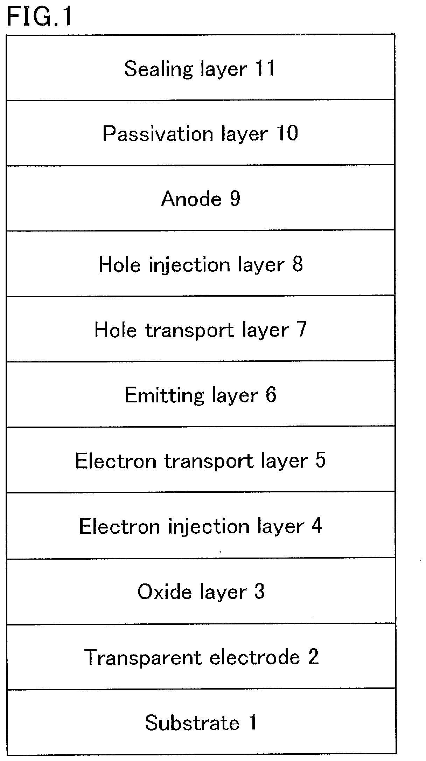

Fig. 1 is a view of a laminated structure of an organic electroluminescence device 1 produced in Example 1.
Fig. 2 is a view showing the voltage-luminance characteristics of the organic electroluminescence device 1 produced in Example 1 to which a direct voltage of 0 V to 5 V is applied in an argon atmosphere.
Fig. 3 is a view showing the current density-current efficiency characteristics of the organic electroluminescence device 1 produced in Example 1 to which a direct voltage of 0 V to 5 V is applied in an argon atmosphere.
Fig. 4 is a view showing the emission spectrum of the organic electroluminescence device 1 produced in Example 1 to which a direct voltage of 0 V to 5 V is applied in an argon atmosphere.
Fig. 5 is a view showing the results of measurement of the temperature of a surface of the organic electroluminescence device 1 produced in Example 1 while the device is continuously driven at a constant luminance of 5,000 cd/m² corresponding to an irradiance of 10 mW/cm².
Fig. 6 is a photograph of a sheet of each of the organic electroluminescence devices produced in the examples and comparative examples with lead-out electrodes.
Fig. 7 is a photograph of the sheet of each of the organic electroluminescence devices produced in the examples and comparative examples with lead-out electrodes, the sheet being directly attached to the skin of a subject.

### DESCRIPTION OF EMBODIMENTS

The present invention is described in detail below.

A combination of two or more of individual preferred embodiments of the present invention described below is also a preferred embodiment of the present invention.

The light cosmetology or light therapy organic electroluminescence sheet of the present invention includes an organic electroluminescence device having a structure in which multiple layers are laminated on a sheet-like flexible substrate and having a thickness of 10 to 100 µm.

Use of a thin and flexible organic electroluminescence device as a light source of a light cosmetology or light therapy sheet can provide a light-emitting sheet having excellent conformability to the skin and excellent wearing feeling.

The organic electroluminescence device in the light cosmetology or light therapy organic electroluminescence sheet preferably has a thickness of 10 to 100 µm, more preferably 10 to 80 µm, still more preferably 10 to 50 µm, particularly preferably 10 to 30 µm.

The thickness of the organic electroluminescence device can be measured with a digital caliper.

### <Organic electroluminescence device>

The organic electroluminescence device in the light cosmetology or light therapy organic electroluminescence sheet of the present invention preferably has a structure in which multiple organic compound layers are laminated between an anode and a cathode. The organic electroluminescence device in the present invention may have any structure, and is preferably a device including a cathode, an electron injection layer and/or an electron transport layer, a light emitting layer, a hole transport layer and/or a hole injection layer, and an anode which are adjacent to each other in this order. Each of these layers may consist of one layer or two or more layers.

When the organic electroluminescence device having the above structure includes either an electron injection layer or an electron transport layer, the layer is laminated adjacent to the cathode and the emitting layer. When the device includes both an electron injection layer and an electron transport layer, the cathode, the electron injection layer, the electron transport layer, and the emitting layer are laminated adjacent to each other in this order. When the device includes either a hole transport layer or a hole injection layer, the layer is laminated adjacent to the emitting layer and the anode. When the device includes both a hole transport layer and a hole injection layer, the emitting layer, the hole transport layer, the hole injection layer, and the anode are laminated adjacent to each other in this order.

The organic electroluminescence device may be a conventional device in which the anode is formed on a sheet-like flexible substrate or may be an inverted device in which the cathode is formed on the substrate. Preferably, the organic electroluminescence device is an inverted device having a structure in which multiple layers are laminated between the cathode on a sheet-like flexible substrate and the anode.

When the light cosmetology or light therapy organic electroluminescence sheet is directly attached to the skin, the device is likely to be affected by water vapor released from the skin. A so-called inverted organic electroluminescence device in which the cathode is formed on the substrate and multiple layers are laminated thereon has a higher resistance to water vapor than a so-called conventional organic electroluminescence device in which the anode is formed on the substrate and multiple layers are laminated thereon. Such an inverted device can achieve a device lifetime required when attached for use to the human body even when the device has a laminated structure including a smaller number of layers or has a thinner sealing portion than the conventional device. Thus, use of an inverted organic electroluminescence device can provide an organic electroluminescence sheet having a required device lifetime, better flexibility, and higher conformability to the skin.

Also, the organic electroluminescence device in the light cosmetology or light therapy organic electroluminescence sheet of the present invention is preferably an organic-inorganic hybrid organic electroluminescence device including a metal oxide layer between the cathode and the anode. Use of a metal oxide as a material of the device can provide an organic electroluminescence device having higher resistance to water vapor.

The organic electroluminescence device in the present invention is preferably an organic-inorganic hybrid organic electroluminescence device including a cathode adjacent to a substrate and a metal oxide layer between an anode and the cathode, further including an emitting layer, the anode, an electron injection layer and an optional electron transport layer between the cathode and the emitting layer, and a hole transport layer and/or a hole injection layer between the anode and the emitting layer. The organic electroluminescence device in the present invention may further include other layers between any of these layers. Still, it preferably consists of only these layers. In other words, a device is preferred in which a cathode, an electron injection layer, an optional electron transport layer, an emitting layer, a hole transport layer and/or a hole injection layer, and an anode are laminated adjacent to each other in this order. Each of these layers may consist of one layer or two or more layers.

In the organic electroluminescence device in the light cosmetology or light therapy organic electroluminescence sheet of the present invention, the emitting layer may be formed from any compound that can be commonly used as a material of an emitting layer. The compound may be a low-molecular compound, a high-molecular compound, or a mixture thereof.

The term "low-molecular material" as used herein means a material that is not a high-molecular material (polymer), and does not necessarily means an organic compound having a low molecular weight.

Examples of the high-molecular material of the emitting layer include polyacetylene compounds such as trans-polyacetylene, cis-polyacetylene, poly(diphenylacetylene) (PDPA), and poly(alkyl,phenylacetylene) (PAPA); polyparaphenylenevinylene compounds such as poly(para-phenylenevinylene) (PPV), poly(2,5-dialkoxy-para-phenylenevinylene) (RO-PPV), cyano-substituted-poly(para-phenylenevinylene) (CN-PPV), poly(2-dimethyloctylsilyl-para-phenylenevinylene) (DMOS-PPV), and poly(2-methoxy,5-(2'-ethylhexoxy)-para-phenylenevinylene) (MEH-PPV); polythiophene compounds such as poly(3-alkylthiophene) (PAT) and poly(oxypropylene)triol (POPT); polyfluorene compounds such as poly(9,9-dialkylfluorene) (PDAF), poly(dioctylfluorene-alt-benzothiadiazole) (F8BT), α,ω-bis[N,N'-di(methylphenyl) aminophenyl]-poly[9,9-bis(2-ethylhexyl)fluorene-2,7-diyl] (PF2/6am4), and poly(9,9-dioctyl-2,7-divinylenefluorenyl-ortho-co(anthracene-9,10-diyl); polyparaphenylene compounds such as poly(para-phenylene) (PPP) and poly(1,5-dialkoxy-para-phenylene) (RO-PPP); polycarbazole compounds such as poly(N-vinylcarbazole) (PVK); polysilane compounds such as poly(methylphenylsilane) (PMPS), poly(naphthylphenylsilane) (PNPS), and poly(biphenylylphenylsilane) (PBPS); and boron compound-based polymer materials disclosed in Japanese Patent Application No. 2010-230995 and Japanese Patent Application No. 2011-6457.

Examples of the low-molecular material of the emitting layer include various metal complexes such as a tridentate iridium complex having 2,2'-bipyridine-4,4'-dicarboxylic acid as a ligand, fac-tris(2-phenylpyridine)iridium (Ir(ppy)₃), 8-hydroxyquinoline aluminum (Alq₃), tris(4-methyl-8-quinolinolate) aluminum(III) (Almq₃), 8-hydroxyquinoline zinc (Znq₂), (1,10-phenanthroline)-tris-(4,4,4-trifluoro-1-(2-thienyl)-butane-1,3-dionate) europium(III) (Eu(TTA)₃(phen)), and 2,3,7,8,12,13,17,18-octaethyl-21H,23H-porphin platinum(II); benzene compounds such as distyrylbenzene (DSB) and diaminodistyrylbenzene (DADSB); naphthalene compounds such as naphthalene and Nile red; phenanthrene compounds such as phenanthrene; chrysene compounds such as chrysene and 6-nitrochrysene; perylene compounds such as perylene and N,N'-bis(2,5-di-t-butylphenyl)-3,4,9,10-perylene-di-carboxy imide (BPPC); coronene compounds such as coronene; anthracene compounds such as anthracene and bisstyrylanthracene; pyrene compounds such as pyrene; pyran compounds such as 4-(di-cyanomethylene)-2-methyl-6-(paradimethylaminostyryl)-4H-pyran (DCM); acridine compounds such as acridine; stilbene compounds such as stilbene; thiophene compounds such as 2,5-dibenzooxazolethiophene; benzooxazole compounds such as benzooxazole; benzimidazole compounds such as benzimidazole; benzothiazole compounds such as 2,2'-(para-phenylenedivinylene)-bisbenzothiazole; butadiene compounds such as bistyryl(1,4-diphenyl-1,3-butadiene) and tetraphenylbutadiene; naphthalimide compounds such as naphthalimide; coumarin compounds such as coumarin; perynone compounds such as perynone; oxadiazole compounds such as oxadiazole; aldazine compounds; cyclopentadiene compounds such as 1,2,3,4,5-pentaphenyl-1,3-cyclopentadiene (PPCP); quinacridone compounds such as quinacridone and quinacridone red; pyridine compounds such as pyrrolopyridine and thiadiazolopyridine; spiro compounds such as 2,2',7,7-tetraphenyl-9,9'-spirobifluorene; metallic or non-metallic phthalocyanine compounds such as phthalocyanine (H₂Pc) and copper phthalocyanine; and boron compound materials disclosed in JP-A 2009-155325, Japanese Patent Application No. 2010-230995, and Japanese Patent Application No. 2011-6458. Products available from Chemipro Kasei Kaisha, Ltd., such as KHLHS-04 and KHLDR-03 may be used, for example.

The emitting layer has any average thickness. The average thickness is preferably 10 to 150 nm, more preferably 20 to 100 nm, still more preferably 40 to 100 nm.

The average thickness of the emitting layer can be measured with a quartz crystal film thickness meter in the case of a low-molecular compound, or with a contact-type step profiler in the case of a high-molecular compound.

The electron transport layer, if present, in the organic electroluminescence device in the light cosmetology or light therapy organic electroluminescence sheet of the present invention may be formed from any of compounds that can be commonly used as a material of an electron transport layer or may be formed from a mixture of these compounds.

Examples of the compounds that can be used as a material of the electron transport layer include pyridine derivatives such as tris-1,3,5-(3'-(pyridin-3"-yl)phenyl) benzene (TmPyPhB); quinoline derivatives such as (2-(3-(9-carbazolyl)phenyl)quinoline (mCQ)); pyrimidine derivatives such as 2-phenyl-4,6-bis(3,5-dipyridylphenyl)pyrimidine (BPyPPM); pyrazine derivatives; phenanthroline derivatives such as bathophenanthroline (BPhen); triazine derivatives such as 2,4-bis(4-biphenyl)-6-(4'-(2-pyridinyl)-4-biphenyl)-[1,3,5]triazine (MPT); triazole derivatives such as 3-phenyl-4-(1'-naphthyl)-5-phenyl-1,2,4-triazole (TAZ); oxazole derivatives; oxadiazole derivatives such as 2-(4-biphenyl)-5-(4-tert-butylphenyl-1,3,4-oxadiazole) (PBD); imidazole derivatives such as 2,2',2"-(1,3,5-benzinetriyl)-tris(1-phenyl-1-H-benzimidazole) (TPBI); aromatic ring tetracarboxylic anhydrides such as naphthalene and perylene; various metal complexes such as bis[2-(2-hydroxyphenyl)benzothiazolato]zinc (Zn(BTZ)₂) and tris(8-hydroxyquinolinato)aluminum (Alq₃); and organic silane derivatives typified by silole derivatives such as 2,5-bis(6'-(2',2"-bipyridyl))-1,1-dimethyl-3,4-diphenylsilole (PyPySPyPy). These may be used alone or in combination of two or more thereof.

Preferred among these are metal complexes such as Alq₃ and pyridine derivatives such as TmPyPhB.

The electron injection layer may be a nitrogen-containing film layer formed from a nitrogen-containing compound.

Examples of the nitrogen-containing compound that forms the nitrogen-containing film layer include compounds containing a nitrogen-containing heterocycle, such as pyrrolidones (e.g., polyvinylpyrrolidone), pyrroles (e.g., polypyrrole), anilines (e.g., polyaniline), pyridines (e.g., polyvinylpyridine), pyrrolidines, imidazoles, piperidines, pyrimidines, and triazines; and amine compounds.

The nitrogen-containing compound is preferably a high nitrogen content compound such as a polyamine. A polyamine, which has a high proportion of the number of nitrogen atoms to the total number of atoms constituting the compound, is suitable to provide an organic electroluminescence device having high electron injection properties and high driving stability.

The polyamine is preferably one that can be formed into a layer by application, and may be a low-molecular compound or a high-molecular compound. The low-molecular compound is preferably a polyalkylene polyamine such as diethylenetriamine or pentamethyldiethylenetriamine. The high-molecular compound is preferably a polymer having a polyalkyleneimine structure. Polyethyleneimine is particularly preferred. In particular, in a preferred embodiment of the present invention, the nitrogen-containing compound is polyethylenimine or diethylenetriamine.

The term "low-molecular compound" herein means a compound that is not a high-molecular compound (polymer), and does not necessarily mean a compound having a low molecular weight.

The nitrogen-containing film may have any average thickness. The average thickness is preferably 0.5 to 10 nm, more preferably 1 to 5 nm, still more preferably 1 to 3 nm.

The average thickness of the emitting layer can be measured with a quartz crystal film thickness meter in the case of a low-molecular compound.

The hole transport layer, if present, in the organic electroluminescence device in the light cosmetology or light therapy organic electroluminescence sheet of the present invention may be formed from an organic material having hole transport properties such as a p-type high-molecular material or a p-type low-molecular material. The p-type high-molecular material and the p-type low-molecular material may be used alone or in combination.

Examples of the p-type high-molecular material (organic polymer) include a polyarylamine, a fluorenearylamine copolymer, a fluorene-bithiophene copolymer, poly(N-vinylcarbazole), polyvinylpyrene, polyvinylanthracene, polythiophene, a polyalkylthiophene, polyhexylthiophene, poly(p-phenylenevinylene), polythienylenevinylene, pyrene-formaldehyde resin, ethylcarbazole-formaldehyde resin, and derivatives thereof. Each of these compounds may be used as a mixture with other compounds. A mixture containing polythiophene may be exemplified by poly(3,4-ethylenedioxythiophene/styrenesulfonate) (PEDOT/PSS).

Examples of the p-type low-molecular material include arylcycloalkane compounds such as 1,1-bis(4-di-paratriaminophenyl) cyclohexane and 1,1'-bis(4-di-paratolylaminophenyl)-4-phenyl-cyclohexane; arylamine compounds such as 4,4',4"-trimethyltriphenylamine, N,N,N',N'-tetraphenyl-1,1'-biphenyl-4,4'-diamine, N,N'-diphenyl-N,N'-bis(3-methylphenyl)-1,1'-biphenyl-4,4'-diamine (TPD1), N,N'-diphenyl-N,N'-bis(4-methoxyphenyl)-1,1'-biphenyl-4,4'-diamine (TPD2), N,N,N',N'-tetrakis(4-methoxyphenyl)-1,1'-biphenyl-4,4'-diamine (TPD3), N,N'-di(1-naphthyl)-N,N'-diphenyl-1,1'-biphenyl-4,4'-diamine (α-NPD), and TPTE; phenylenediamine compounds such as N,N,N',N'-tetraphenylpara-phenylenediamine, N,N,N',N'-tetra(para-tolyl)-paraphenylenediamine, and N,N,N',N'-tetra(meta-tolyl)-metaphenylenediamine (PDA); carbazole compounds such as carbazole, N-isopropylcarbazole, and N-phenylcarbazole; stilbene compounds such as stilbene and 4-di-paratolylaminostilbene; oxazole compounds such as OₓZ; triphenylmethane compounds such as triphenylmethane and m-MTDATA; pyrazoline compounds such as 1-phenyl-3-(paradimethylaminophenyl) pyrazoline; benzine(cyclohexadiene) compounds; triazole compounds such as triazole; imidazole compounds such as imidazole; oxadiazole compounds such as 1,3,4-oxadiazole and 2,5-di(4-dimethylaminophenyl)-1,3,4-oxadiazole; anthracene compounds such as anthracene and 9-(4-diethylaminostyryl)anthracene; fluorenone compounds such as fluorenone, 2,4,7-trinitro-9-fluorenone, and 2,7-bis(2-hydroxy-3-(2-chlorophenylcarbamoyl)-1-naphthylazo) fluorenone; aniline compounds such as polyaniline; silane compounds; pyrrole compounds such as 1,4-dithioketo-3,6-diphenyl-pyrrolo-(3,4-c)pyrrolopyrrole; fluorene compounds such as fluorene; porphyrin compounds such as porphyrin and metal tetraphenylporphyrin; quinacridon compounds such as quinacridon; metallic or non-metallic phthalocyanine compounds such as phthalocyanine, copper phthalocyanine, tetra(t-butyl)copper phthalocyanine, and iron phthalocyanine; metallic or non-metallic naphthalocyanine compounds such as copper naphthalocyanine, vanadyl naphthalocyanine, and monochloro gallium naphthalocyanine; and benzidine compounds such as N,N'-di(naphthalen-1-yl)-N,N'-diphenyl-benzidine and N,N,N',N'-tetraphenylbenzidine.

The electron transport layer and the hole transport layer, if present, in the organic electroluminescence device in the light cosmetology or light therapy organic electroluminescence sheet of the present invention may each have any average thickness. The average thickness is preferably 10 to 150 nm, more preferably 20 to 100 nm, still more preferably 40 to 100 nm.

The average thicknesses of the electron transport layer and the hole transport layer can be measured with a quartz crystal film thickness meter in the case of a low-molecular compound, or with a contact-type step profiler in the case of a high-molecular compound.

The metal oxide layer, if present, in the organic electroluminescence device in the light cosmetology or light therapy organic electroluminescence sheet of the present invention is present between the cathode and the emitting layer and/or between the anode and the emitting layer. Preferably, the metal oxide layer is present both between the cathode and the emitting layer and between the emitting layer and the anode. When the metal oxide layer between the cathode and the emitting layer is expressed as a first metal oxide layer and the metal oxide layer between the anode and the emitting layer is expressed as a second metal oxide layer, the first metal oxide layer preferably serves as an electron injection layer and the second metal oxide layer preferably serves as a hole injection layer. A preferred example of the structure of the organic electroluminescence device in the present invention is a structure in which a cathode, the first metal oxide layer, a nitrogen-containing film layer, an emitting layer, a hole transport layer, the second metal oxide layer, and an anode are laminated adjacent to each other in this order. An electron transport layer may optionally be present between the nitrogen-containing film layer and the emitting layer. As for the importance of the metal oxide layers, the first metal oxide layer is more important than the second metal oxide layer, and the second metal oxide layer may be replaced by, for example, HATCN or another organic material having an extremely deep lowest unoccupied molecular orbital level.

The first metal oxide layer is a layer of a thin semiconductive or insulating film consisting of one single-metal oxide film, or a layer of thin semiconductive or insulating films consisting of a laminate and/or a mixture of single-metal oxides or multiple-metal oxides. The metal element of the metal oxide is selected from the group consisting of magnesium, calcium, strontium, barium, titanium, zirconium, hafnium, vanadium, niobium, tantalum, chromium, molybdenum, tungsten, manganese, indium, gallium, iron, cobalt, nickel, copper, zinc, cadmium, aluminum, and silicon. The layer consisting of a laminate or a mixture of metal oxides preferably includes a layer formed from at least one metal element selected from magnesium, aluminum, calcium, zirconium, hafnium, silicon, titanium, or zinc among the metal elements mentioned above. In the case of the layer consisting of single-metal oxides, the layer preferably includes a metal oxide selected from the group consisting of magnesium oxide, aluminum oxide, zirconium oxide, hafnium oxide, silicon oxide, titanium oxide, and zinc oxide.

Examples of the layer consisting of a laminate and/or a mixture of single-metal oxides or multiple-metal oxides include layers in which the following combinations of metal oxides are laminated and/or mixed: titanium oxide/zinc oxide, titanium oxide/magnesium oxide, titanium oxide/zirconium oxide, titanium oxide/aluminum oxide, titanium oxide/hafnium oxide, titanium oxide/silicon oxide, zinc oxide/magnesium oxide, zinc oxide/zirconium oxide, zinc oxide/hafnium oxide, zinc oxide/silicon oxide, calcium oxide/aluminum oxide, and the like. Examples thereof also include layers in which the following combinations of three kinds of metal oxides are laminated and/or mixed: titanium oxide/zinc oxide/magnesium oxide, titanium oxide/zinc oxide/zirconium oxide, titanium oxide/zinc oxide/aluminum oxide, titanium oxide/zinc oxide/hafnium oxide, titanium oxide/zinc oxide/silicon oxide, indium oxide/gallium oxide/zinc oxide, and the like. The above examples also include IGZO, an oxide semiconductor, and 12CaO•7Al₂O₃, an electroride, which have special compositions and exhibit good properties.

The first metal oxide layer also serves as an electron injection layer or an electrode (cathode).

In the present invention, those having a sheet resistance of less than 1000/□ are classified as conductors, and those having a sheet resistance of not less than 100Ω/□ are classified as semiconductors or insulators. Thus, thin films known as transparent electrodes such as tin-doped indium oxide (ITO), antimony-doped indium oxide (ATO), indium-doped zinc oxide (IZO), aluminum-doped zinc oxide (AZO), and fluorine-doped indium oxide (FTO) thin films, which are highly conductive, do not fall into the category of the semiconductor or insulator and therefore do not meet the definition of a film constituting the first metal oxide layer.

The first metal oxide layer may be any laminate containing a metal oxide layer and an elemental metal layer as long as the first metal oxide layer contains a metal oxide layer.

The metal oxide is constituted by any of the elements described above.

Examples of metal in the elemental metal layer include silver and palladium. The elemental metal layer may contain one or more of these.

When the first metal oxide layer is a laminate containing a metal oxide layer and an elemental metal layer, the metal oxide layer and the elemental metal layer are preferably alternately laminated. In this case, preferably, the number of the metal oxide layers is two and the number of the elemental metal layers is one. In other words, a structure in which one elemental metal layer is present between two metal oxide layers is preferred.

The second metal oxide layer may be formed from any metal oxide, and examples thereof include vanadium oxide (V₂O₅), molybdenum oxide (MoO₃), tungsten oxide (WO₃), and ruthenium oxide (RuO₂). These may be used alone or in combination of two or more thereof. Among these, vanadium oxide or molybdenum oxide is preferably used as a main component. The second metal oxide layer containing vanadium oxide or molybdenum oxide as a main component has an excellent function as a hole injection layer, i.e., an ability to inject holes from the anode and transport the holes to the emitting layer or the hole transport layer. Also, vanadium oxide and molybdenum oxide inherently have high hole-transporting properties and thus have advantages that they can suitably prevent a decrease in injection efficiency of holes from the anode to the emitting layer or the hole transport layer. More preferably, the second metal oxide layer is formed from vanadium oxide and/or molybdenum oxide.

The first metal oxide layer may have an average thickness ranging from 1 nm to about several micrometers. In order to obtain an organic electroluminescence device that can operate at low voltages, the average thickness is preferably 1 to 1000 nm, more preferably 2 to 100 nm.

The second metal oxide layer may have any average thickness. The average thickness is preferably 1 to 1000 nm, more preferably 5 to 50 nm.

The average thickness of the first metal oxide layer can be measured with a probe-type step profiler or by spectroscopic ellipsometry.

The average thickness of the second metal oxide layer can be measured with a quartz crystal film thickness meter during film formation.

In the organic electroluminescence device in the light cosmetology or light therapy organic electroluminescence sheet of the present invention, the anode and the cathode each may be formed from a known appropriate conductive material. In order to extract light, at least one of them is preferably transparent. Examples of a known transparent conductive material include tin-doped indium oxide (ITO), antimony-doped indium oxide (ATO), indium-doped zinc oxide (IZO), aluminum-doped zinc oxide (AZO), and fluorine-doped indium oxide (FTO). Examples of an opaque conductive material include calcium, magnesium, aluminum, tin, indium, copper, silver, gold, and platinum, and alloys thereof.

The cathode is preferably ITO, IZO, or FTO among these.

The anode is preferably Au, Ag, or Al among these. Since metals commonly used for an anode can be used for the cathode and the anode as described above, a top emission structure in which light is extracted from the upper electrode can be readily achieved, and each electrode can be selected from the above various types of electrodes. For example, Al may be used for the lower electrode and ITO may be used for the upper electrode.

The cathode may have any average thickness. The average thickness is preferably 10 to 500 nm, more preferably 100 to 200 nm. The average thickness of the cathode can be measured with a probe-type step profiler or by spectroscopic ellipsometry.

The anode may have any average thickness. The average thickness is preferably 10 to 1000 nm, more preferably 30 to 150 nm. An opaque material can be used as an anode for the top emission type device and the transparent type device when the average thickness of the opaque material is about 10 to 30 nm.

The average thickness of the anode can be measured with a quartz crystal film thickness meter during film formation.

In the organic electroluminescence device in the present invention, the organic compound layers may be formed by any method. An appropriate method may be selected from various methods according to the properties of the material. When the material can be applied in the form of a solution, various application methods can be employed such as spin coating, casting, micro gravure coating, gravure coating, bar coating, roll coating, wire bar coating, slit coating, dip coating, spray coating, screen printing, flexographic printing, offset printing, and inkjet printing. Preferred among these are spin coating and slit coating because they easily control the thickness of a layer. When the material is not applied or is less soluble in a solvent, vacuum deposition and evaporative spray deposition from ultra-dilute solution (ESDUS) are suitable, for example.

When the organic compound layers are each formed by applying an organic compound solution, inorganic solvents and various organic solvents can be used to dissolve the organic compound. Examples of the inorganic solvents include nitric acid, sulfuric acid, ammonia, hydrogen peroxide, water, carbon disulfide, carbon tetrachloride, and ethylene carbonate. Examples of the organic solvents include ketone solvents such as methyl ethyl ketone (MEK), acetone, diethyl ketone, methyl isobutyl ketone (MIBK), methyl isopropyl ketone (MIPK), and cyclohexanone; alcohol solvents such as methanol, ethanol, isopropanol, ethylene glycol, diethylene glycol (DEG), and glycerine; ether solvents such as diethyl ether, diisopropylether, 1,2-dimethoxyethane (DME), 1,4-dioxane, tetrahydrofuran (THF), tetrahydropyran (THP), anisole, diethylene glycol dimethyl ether (diglyme), and diethylene glycol ethyl ether (carbitol); cellosolve solvents such as methyl cellosolve, ethyl cellosolve, and phenyl cellosolve; aliphatic hydrocarbon solvents such as hexane, pentane, heptane, and cyclohexane; aromatic hydrocarbon solvents such as toluene, xylene, and benzene; aromatic heterocyclic compound solvents such as pyridine, pyrazine, furan, pyrrole, thiophene, and methylpyrrolidone; amide solvents such as N,N-dimethylformamide (DMF) and N,N-dimethylacetamide (DMA); halogenated compound solvents such as chlorobenzene, dichloromethane, chloroform, and 1,2-dichloroethane; ester solvents such as ethyl acetate, methyl acetate, and ethyl formate; sulfur compound solvents such as dimethyl sulfoxide (DMSO) and sulfolane; nitrile solvents such as acetonitrile, propionitrile, and acrylonitrile; and organic acid solvents such as formic acid, acetic acid, trichloroacetic acid, and trifluoroacetic acid. Examples also include mixtures of these solvents.

Preferred among these solvents are non-polar solvents, and examples thereof include aromatic hydrocarbon solvents such as xylene, toluene, cyclohexylbenzene, dihydrobenzofuran, trimethylbenzene, and tetramethylbenzene, aromatic heterocyclic compound solvents such as pyridine, pyrazine, furan, pyrrole, thiophene, and methylpyrrolidone, and aliphatic hydrocarbon solvents such as hexane, pentane, heptane, and cyclohexane. These may be used alone or as a mixture of two or more thereof.

The cathode, the anode, and the oxide layer can be formed by a method such as a sputtering method, a vacuum deposition method, a sol-gel method, a spray pyrolysis deposition (SPD) method, an atomic layer deposition (ALD) method, a vapor phase deposition method, or a liquid phase deposition method. The cathode and the anode may also be formed by joining metal foil. A method suitable for each layer may be selected from these methods according to the properties of the material of the layer. These layers may be formed by different methods. The second metal oxide layer is more preferably formed by vapor phase deposition among these methods. The vapor phase deposition enables clean formation of the second metal oxide layer without destroying the surface of the organic compound layer and in good contact with the anode. As a result, the effects owing to the presence of the second metal oxide layer as described above are more significantly achieved.

In order to further enhance the properties of the organic electroluminescence device, the organic electroluminescence device may further include, if necessary, a hole blocking layer and an electron blocking layer, for example. These layers may be formed from materials commonly used to form these layers and may be formed by a method commonly used to form these layers.

The organic electroluminescence device may further include a passivation layer on the last-formed electrode of the laminated structure so as to protect the surface. The passivation layer may be formed from any material commonly used to form a passivation layer. For example, the material may include the materials for the hole transport layer and/or the materials for the metal oxide layer a described above, but is not limited to these as long as the material includes a combination capable of keeping insulation.

The passivation layer may have any average thickness. The average thickness is preferably 20 to 300 nm, more preferably 50 to 200 nm.

The average thickness of the passivation layer can be measured with a quartz crystal film thickness meter during film formation.

The organic electroluminescence device includes a sheet-like flexible substrate.

Examples of materials of the substrate include resin materials such as polyethylene terephthalate, polyethylene naphthalate, polypropylene, a cycloolefin polymer, a polyamide, polyethersulfone, polymethyl methacrylate, polycarbonate, and polyacrylate.

The flexible substrate may be one in which a surface of the resin material is coated with epoxy resin or the like.

The substrate preferably has an average thickness of 10 to 150 µm, more preferably 10 to 50 µm.

The average thickness of the substrate can be measured with a digital caliper.

The organic electroluminescence device may further include a sealing layer on the passivation layer. The sealing layer may be formed from the same material as that of the sheet-like flexible substrate, and the thickness of the sealing layer is preferably the same as the thickness of the sheet-like flexible substrate.

When the organic electroluminescence device in the light cosmetology or light therapy organic electroluminescence sheet of the present invention is sealed, it may be appropriately sealed by a common method. Examples of the method include bonding a sealing container in an inert gas and forming a sealing film directly on the organic electroluminescence device. Sealing the organic electroluminescence device with a water absorber may be used together with these methods.

Although the aforementioned inverted organic electroluminescence device requires less strict sealing than a conventional organic electroluminescence device, it may be sealed as needed.

The organic electroluminescence device may be a top emission device in which light is emitted from the side opposite to the substrate, or may be a bottom emission device in which light is emitted from the substrate side.

### <Thin film battery>

The light cosmetology or light therapy organic electroluminescence sheet of the present invention may be connected to an external power source when used or may contain a thin film battery therein. An organic electroluminescence sheet containing a thin film battery can be used without having a connection with an external power source, and thus has higher portability and the like and is more convenient to use.

In a preferred embodiment of the light cosmetology or light therapy organic electroluminescence sheet of the present invention, the organic electroluminescence sheet further contains a thin film battery.

The thin film battery may be a thin film primary battery or secondary battery, and may be a thin film lithium-ion battery containing a solid electrolyte, for example.

Non-limiting examples of a positive electrode active material of the thin film battery include lithium composite oxides such as LiCoO₂, LiMnO₂, LiFePO₄, and LiCoPO₄ and metallic sulfides free from lithium, such as TiS and MoS₂. The positive electrode active material may include one or more of these.

Non-limiting examples of a negative electrode active material of the thin film battery include carbon materials such as graphite; metal oxides such as V₂O₅; and materials capable of forming alloys with lithium, such as an elemental Si or Sn and an alloy and a compound of Si or Sn. The negative electrode active material may include one or more of these.

Non-limiting examples of a solid electrolyte of the thin film battery include lithium nitride, lithium halides, and lithium phosphates, such as Li₃N, LiI, Li₃PO₄, and LiPO₄₋ₓNₓ and lithium-containing sulfide glass solid electrolytes such as Li₂s-SiS₂, Li₂S-P₂S₅, and Li₂S-B₂S₃. The solid electrolyte may include one or more of these.

The thin film battery preferably has a thickness of 450 µm or smaller. Use of a thin film battery having such a thickness can reduce the thickness of the organic electroluminescence sheet of the present invention. Such an organic electroluminescence sheet has much better conformability to the skin and causes less discomfort when it is attached to the skin. The thickness of the thin film battery is more preferably 100 to 300 µm, still more preferably 50 to 100 µm.

The thickness of the thin film battery can be measured with a digital multimeter or a caliper.

The thin film battery may be produced by, but not limited to, laminating positive electrode, solid electrolyte, and negative electrode layers on a substrate by sputtering.

### <Other components>

The light cosmetology or light therapy organic electroluminescence sheet of the present invention may optionally contain components other than the organic electroluminescence device and the thin film battery. Examples of the components include a sticking layer or an adhesive layer between the skin and the sheet, a color filter layer for control of wavelength, a buffer layer for enhancement of light emitting, wiring and a power switch for electrical connection between electronic components, and a package for appearance. The light cosmetology or light therapy organic electroluminescence sheet of the present invention may contain one or more of these components.

The light cosmetology or light therapy organic electroluminescence sheet of the present invention preferably has a thickness of 650 µm or smaller. As described above, the light cosmetology or light therapy organic electroluminescence sheet of the present invention may contain components other than the organic electroluminescence device. When such a sheet has a thickness of 650 µm or smaller as a whole, it has much better conformability to the skin and causes less discomfort when it is attached to the skin. The thickness of the organic electroluminescence sheet is more preferably 10 to 600 µm, still more preferably 50 to 100 µm.

The thickness of the organic electroluminescence sheet can be measured with a digital multimeter or a caliper.

Since the light cosmetology or light therapy organic electroluminescence sheet of the present invention has high conformability to the skin and has a long device lifetime, it is suitable for light cosmetology applications for removing blotches and wrinkles on the skin and for medical treatment applications for treating injuries and diseases.

The present invention also relates to a light cosmetology instrument and a light therapy instrument each containing the light cosmetology or light therapy organic electroluminescence sheet of the present invention.

### EXAMPLES

The present invention is described in more detail with reference to examples below, but the present invention is not limited to these examples. Herein, "part(s)" means "part(s) by weight" and "%" means "% by mass" unless otherwise stated.

### 1. Preparation of organic electroluminescence device

### (Example 1)

An organic electroluminescence device 1 was produced in the following way.

### [Step 1]

A substrate 1 was prepared in such a way that SU-8 available from Nippon Kayaku Co., Ltd. was applied by spin coating to a 25-µm-thick PET film with a barrier layer purchased from Oike & Co., Ltd. and was baked on an electric griddle heated to 100°C

### [Step 2]

The substrate 1 was set on a sputtering apparatus, and on the substrate 1 was formed a zinc oxide (ZnO) layer having an average thickness of 20 nm by sputtering using a zinc metal target, oxygen as a reactant gas, and argon as a carrier gas. Thereafter, the substrate 1 was set on a vacuum deposition apparatus, and a silver layer having an average thickness of 8 nm was formed thereon. The substrate 1 was set on the sputtering apparatus again, and a zinc oxide layer having an average thickness of 2 nm was formed thereon. The substrate 1 was placed in the atmosphere and annealed with the electric griddle at 100°C for 30 minutes. Thus, a transparent electrode 2 and an oxide layer 3 were formed.

### [Step 3]

Then, polyethylenimine available from Nippon Shokubai Co., Ltd. was applied to the oxide layer 3 by spin coating to form an electron injection layer 4. The electron injection layer 4 had an average thickness of 2 nm.

### [Step 4]

Then, the substrate 1 on which the layers up to the electron injection layer 4 were formed was fixed on a substrate holder of the vacuum deposition apparatus. Also, KHLHS-04 and KHLDR-03 purchased from Chemipro Kasei Kaisha, Ltd. and N,N'-di(1-naphthyl)-N,N'-diphenyl-1,1'-biphenyl-4,4'-diamine (α-NPD) represented by the formula (1) below were put into different alumina crucibles, and the crucibles were set on a deposition source. The pressure in the vacuum deposition apparatus was reduced to about 1 × 10⁻⁵ Pa, and KHLHS-04 was deposited to 10 nm to form an electron transport layer 5. Then, KHLHS-04 and α-NPD as host materials and KHLDR-03 as an emitting dopant were codeposited to 15 nm to form an emitting layer 6. Next, α-NPD was deposited to 40 nm to form a hole transport layer 7. Further, a film of molybdenum trioxide MoO₃ was formed by in-situ vacuum processing to form a 10-nm-thick hole injection layer 8.

### [Step 5]

Next, aluminum (anode 9) was deposited to a thickness of 70 nm on the substrate 1 on which the layers up to the hole injection layer 8 were formed.

### [Step 6]

Then, α-NPD was deposited to 100 nm on the substrate 1 on which the layers up to the anode 9 were formed, followed by forming a 20-nm-thick zinc oxide film with a sputtering apparatus. Thus, a passivation layer 10 was obtained.

### [Step 7]

Then, the substrate 1 on which the layers up to the passivation layer 10 were formed was transferred to a glove box, and on the substrate 1 were laminated TB1655 available from ThreeBond Co., Ltd. and a 25-µm-thick PET film with a barrier layer purchased from Oike & Co., Ltd. The workpiece was annealed for one hour on an electric griddle heated to 90°C to form a sealing layer 11. Thus, a "device 1" was obtained as an example of the present invention.

A digimatic indicator ID-C112AB available from Mitutoyo Corporation was used to measure the thickness of the device 1, and the total thickness was found to be 72 µm.

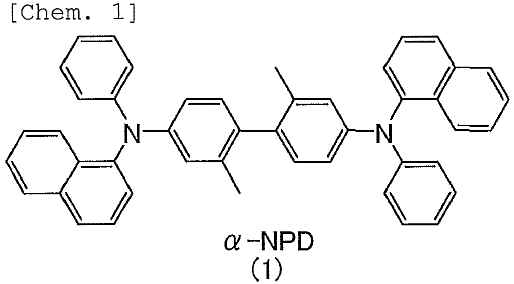

### (Example 2)

An organic electroluminescence device was produced as in Example 1 except that [Step 7] was skipped.

### (Example 3)

An organic electroluminescence device was produced as in Example 1 except that in [Step 1], a 50-µm-thick PET film with a barrier layer was used instead of the 25-µm-thick PET film with a barrier layer.

### (Comparative Example 1)

An organic electroluminescence device was produced as in Example 1 except that in [Step 1] and [Step 7], a 50-µm-thick PET film with a barrier layer was used instead of the 25-µm-thick PET film with a barrier layer.

### (Comparative Example 2)

An organic electroluminescence device was produced as in Example 1 except that in [Step 1] and [Step 7], a 75-µm-thick PET film with a barrier layer was used instead of the 25-µm-thick PET film with a barrier layer.

### (Organic electroluminescence devices produced)

The total thicknesses of the organic electroluminescence devices produced in Examples 1 to 3 and Comparative Examples 1 and 2 measured with a digimatic indicator ID-C112AB available from Mitutoyo Corporation are shown in Table 1.

**[Table 1]**

| | Total thickness µm) |
|---|---|
| Example 1 | 72 |
| Example 2 | 25 |
| Example 3 | 97 |
| Comparative Example 1 | 122 |
| Comparative Example 2 | 172 |

### 2. Measurement of luminescence properties of organic electroluminescence device

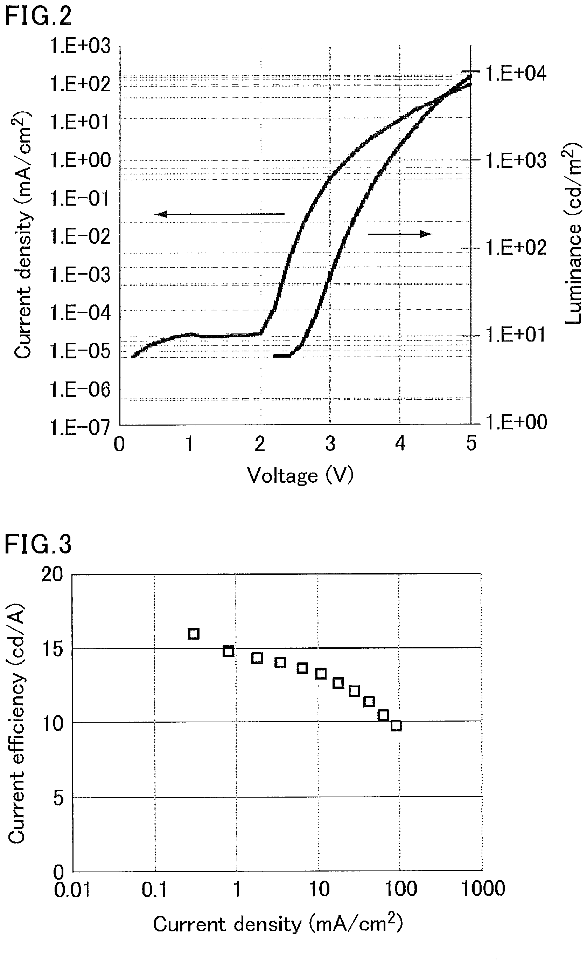

"Series 2400 SourceMeter" available from Keithley Instruments was used to apply a voltage to the device and to measure the current. "BM-7" and a spectroradiometer "SR-3" available from Topcon Corporation were used to measure the light emission luminance and EL spectrum. A direct voltage of 0 V to 5 V was applied to the organic electroluminescence device produced in Example 1 in an argon atmosphere, and the voltage-luminance characteristics, the current density-current efficiency characteristics, and the EL spectrum of the device were measured. They are respectively shown in Figs. 2, 3, and 4. The devices produced in Examples 2 and 3 and Comparative Examples 1 and 2 were subjected to the same measurements and found to have the similar characteristics as those of the device of Example 1.

### 3. Measurement of change in surface temperature of organic electroluminescence device during continuous driving at constant luminance

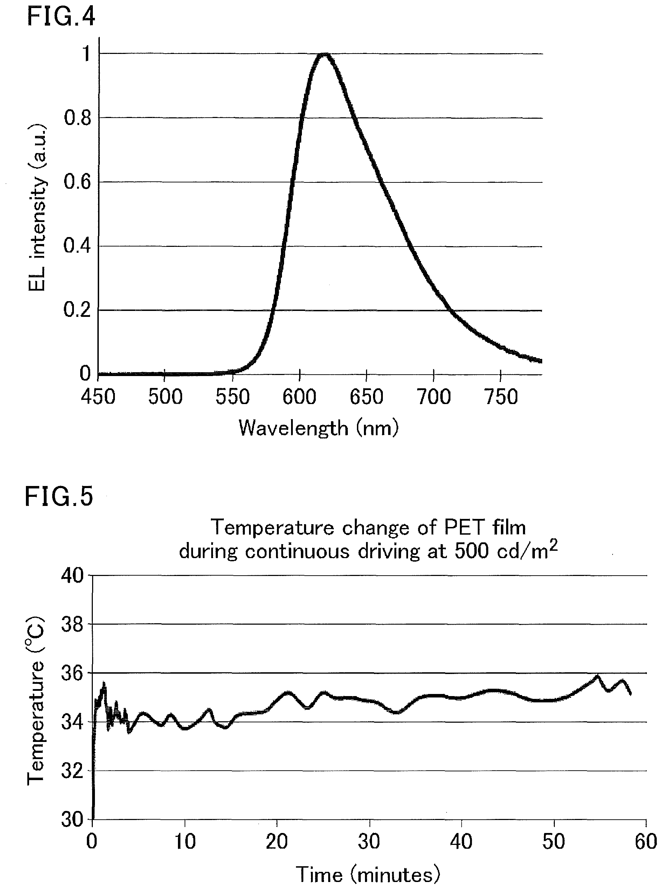

Since organic electroluminescence devices are devices generating heat, they may cause low temperature burn when used in direct contact with the skin. The device produced in Example 1 is to be used closest to the skin among the devices produced in Examples 1 to 3 and Comparative Examples 1 and 2, and thus may involve a risk of low temperature burn. Such an organic electroluminescence device produced in Example 1 was subjected to measurement of the temperature of a surface to be in contact with the skin while the device was continuously driven at a constant luminance of 5,000 cd/m² corresponding to an irradiance of 10 mW/cm² that was assumed to be actually used. The measurement was performed using an infrared thermography camera (R300SR) available from Nippon Avionics Co., Ltd. Fig. 5 shows the resulting data, which demonstrates that the surface temperature did not exceed 36°C even after long-time use, and there is no concern about low temperature burns.

### 4. Evaluation test of organic electroluminescence sheet

The conformability and adhesion of the organic electroluminescence sheet to the skin were evaluated in the following way.

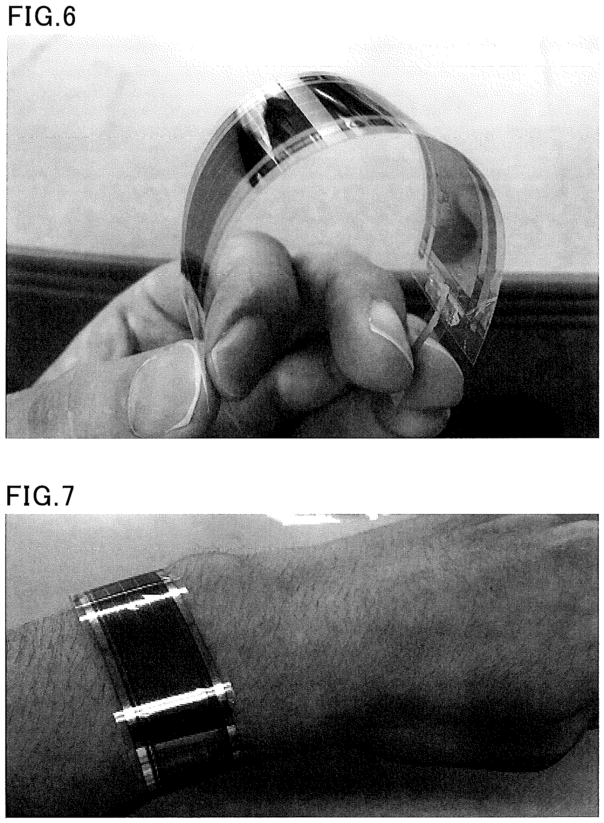

Each of the organic electroluminescence sheets produced in Examples 1 to 3 and Comparative Examples 1 and 2 was provided with lead-out electrodes as shown in Fig. 6, and was worn directly on the skin of a subject as shown in Fig. 7. The lead-out electrodes were connected to an external electrode, and light was continuously emitted for one hour. The subject was randomly selected regardless of gender and age.

### (Test of conformability to the skin (sensory test))

As the indexes of the conformability of the attached organic electroluminescence sheet, the discomfort at and movability of a portion (mainly derived from tightness or restrained feeling at the portion) to which the sheet was attached were evaluated by the following criteria. The evaluation was performed by ten subjects. The results are averaged and shown in Table 2.

### [Evaluation criteria]

5: Movable without any wearing discomfort
4: Slightly movable with slight wearing discomfort
3: Not evaluable
2: Slightly difficult to move with wearing discomfort
1: Difficult to move with high wearing discomfort

### (Test of adhesion to the skin)

The organic electroluminescence sheet was attached to the skin and visually observed to examine the occurrence and degree of lifting and peeling of the sheet within one hour. As the indexes of the conformability, adhesion to the skin and peelability were evaluated by the following criteria. The evaluation was performed by ten subjects. The results are averaged and shown in Table 2.

### [Evaluation criteria]

5: The sheet adhered to the skin, with no lifting and peeling visually observed.
4: Lifting and peeling were visually observed, and the peeled area was less than 5%.
3: Lifting and peeling were visually observed, and the peeled area was not less than 5% and less than 10%.
2: Lifting and peeling were visually observed, and the peeled area was not less than 10% and less than 20%.
1: Lifting and peeling were visually observed frequently, and adhesion was poor.

**[Table 2]**

| | | Example 1 | Example 2 | Example 3 | Comparative Example 1 | Comparative Example 2 |
|---|---|---|---|---|---|---|
| Evaluation | Test of conformability to skin | 4 | 5 | 4 | 3 | 1 |
| | Test of adhesion to skin | 5 | 5 | 5 | 3 | 1 |

As shown in Table 2, the organic electroluminescence sheets of the examples had good conformability and adhesion to the skin and reduced discomfort during wearing. Thus, they are suitable for long time use, for example.

### 5. Evaluation of effect of light emitted by organic electroluminescence device on gene expression

In order to evaluate the effect of the device 1 produced in Example 1 on expression of a variety of genes by DNA microarray analysis using normal human epidermal cells, the following in vitro utility evaluation test (test for evaluating gene expression using epidermal cells) was performed.

Table 3 shows the concentrations and RIN values of RNAs extracted from normal human epidermal cells treated to be made into test samples, and Tables 4 and 5 show the results of the microarray analysis.

### <Test method>

Normal human epidermal cells were inoculated on HuMedia-KG2 in a 12-well plate with a cell density of 2.5 × 10⁵ cells/well. After 24-hour incubation, all the mediums were replaced with a Hanks buffer solution (free from Ca²⁺ and Mg²⁺). Light having an irradiance of 2.5 mW/cm², light having an irradiance of 5 mW/cm², and light having an irradiance of 1 mW/cm² were applied for 60 minutes, for 30 minutes, and for 60 minutes, respectively, using the organic electroluminescence device. The area to be used as a non-exposed area was protected from light with aluminum foil. After the light application, the mediums were replaced with HuMedia-KB2, and incubation was performed for 24 hours. After 24 hours, the cells were immersed and lysed in QIAzol^{(R)} reagent. Purified RNA was extracted from the solution using miRNeasy^{(R)} Mini Kit (QIAGEN). The extracted RNA was subjected to DNA microarray analysis using a chip for mRNA expression analysis (DNA chip genopal^{(R)} NDR custom chip). The results were analyzed, and the results of the analysis of expression of a variety of genes were expressed as ratios relative to the correction value of the control not treated to be made into a test sample, taken as 1, and the significance test was performed.

### [Details of test]

- The number of test samples was n = 3.
- Three 12-well plates were used for the test. Each plate was divided into four areas: a "non-exposed area", an area exposed to light having an "irradiance of 2.5 mW/cm² for 60 minutes", an area exposed to light having an "irradiance of 5 mW/cm² for 30 minutes", and an area exposed to light having an "irradiance of 1 mW/cm² for 60 minutes". This exposure was repeated for the three plates.

- The time for which the cells were immersed in the Hanks buffer solution was the same among the non-exposed area and the exposed areas.
- Light was emitted from a light source placed on the lid of each 12-well plate.

**[Table 3]**

| Conditions | Total RNA concentration (mg/µL) | RIN |
|---|---|---|
| Non-exposed area | 0.36 | 10.0 |
| | 0.41 | 9.9 |
| | 0.36 | 9.9 |
| Exposed to light having irradiance of 2.5 mW/cm² for 60 minutes | 0.34 | 9.8 |
| | 0.39 | 9.9 |
| | 0.38 | 9.9 |
| Exposed to light having irradiance of 5 mW/cm² for 30 minutes | 0.38 | 9.9 |
| | 0.39 | 9.9 |
| | 0.41 | 9.9 |
| Exposed to light having irradiance of 1 mW/cm² for 60 minutes | 0.35 | 9.9 |
| | 0.39 | 9.8 |
| | 0.43 | 9.8 |

Table 3 demonstrated that the concentrations of RNAs extracted were similar among the areas regardless of application of light, and the RIN values (0 (RNA greatly decomposed) to 10 (RNA did not decompose)) were high.

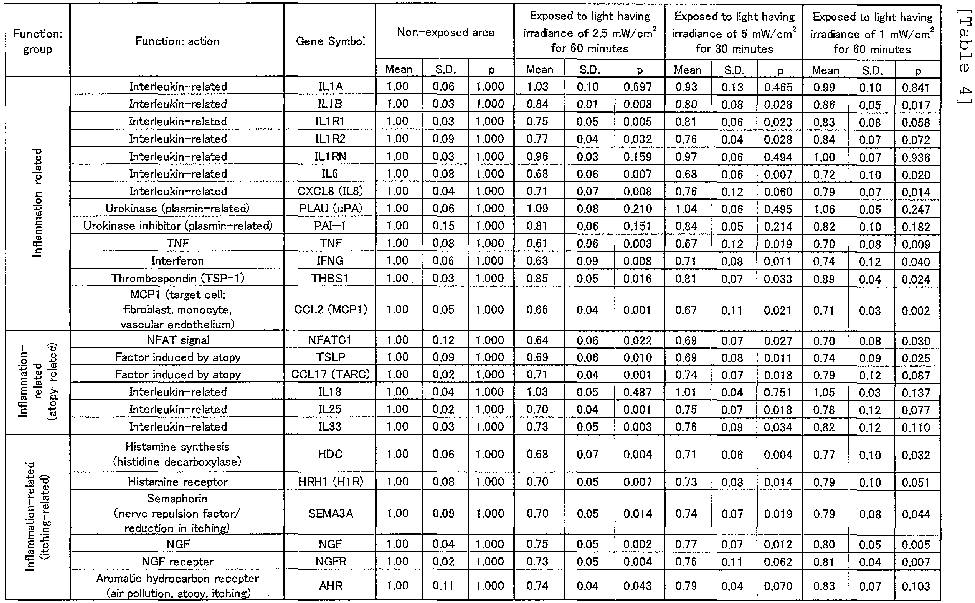

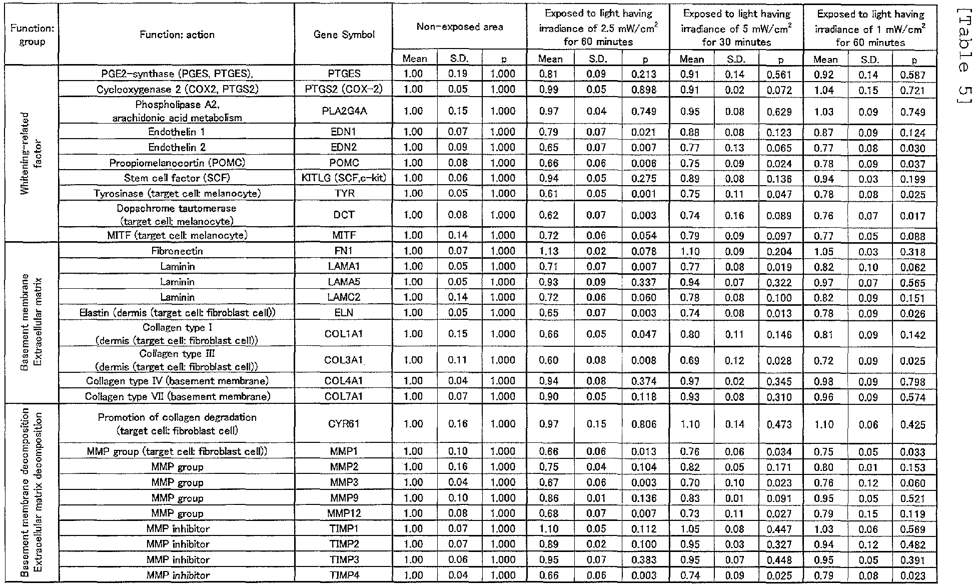

The results of the microarray analysis shown in Tables 4 and 5 demonstrated that expressions of many types of genes were reduced under all the three different conditions where light was applied.

The light application did not cause a great change in the amounts of extracted RNAs and the RIN values, which indicates that the light application does not induce cell damage with RNA decomposition. This suggests that the light application under the conditions may reduce gene expression.

Under the conditions where no cell damage is considered to be induced, gene expression of inflammatory factors, factors that induce pigmentation, or matrix metalloproteinase was reduced. This suggests that the light application may achieve an anti-inflammatory action.

## Claims

1. A light cosmetology or light therapy organic electroluminescence sheet used in light cosmetology or light therapy application, the organic electroluminescence sheet comprising:
an organic electroluminescence device having a structure in which multiple layers are laminated on a sheet-like flexible substrate and having a thickness of 10 to 100 µm.

2. The light cosmetology or light therapy organic electroluminescence sheet according to claim 1,
wherein the organic electroluminescence device comprises a structure in which the multiple layers are laminated between a cathode on the sheet-like flexible substrate and an anode.

3. The light cosmetology or light therapy organic electroluminescence sheet according to claim 2,
wherein the organic electroluminescence device comprises a metal oxide layer between the cathode on the sheet-like flexible substrate and the anode.

4. The light cosmetology or light therapy organic electroluminescence sheet according to any one of claims 1 to 3, further comprising a thin film battery.

5. The light cosmetology or light therapy organic electroluminescence sheet according to any one of claims 1 to 4,
wherein the organic electroluminescence sheet has a thickness of 650 µm or smaller.

6. A light cosmetology instrument comprising:
the light cosmetology or light therapy organic electroluminescence sheet according to any one of claims 1 to 5.

7. A light therapy instrument comprising:
the light cosmetology or light therapy organic electroluminescence sheet according to any one of claims 1 to 5.
